# EUROPÄISCHE PATENTANMELDUNG

(11) **EP 2 465 934 A1**
(43) Veröffentlichungstag der Anmeldung: **20.06.2012**
(21) Anmeldenummer: 10195963.3
(22) Anmeldetag: 20.12.2010
(51) Int. Cl.: C12N 15/10, C12Q 1/68

(54) **Extraktion von Nukleinsäuren**

(71) Anmelder: Mergemeier, Steffen, 10243 Berlin (DE)
(72) Erfinder: Mergemeier, Steffen, 10243 Berlin (DE)
(74) Vertreter: Lange, Sven

(57) **Zusammenfassung**

Die Erfindung beschreibt eine Nukleinsäuren-Extraktionsmaterial, welches mindestens ein Ionenaustauschmaterial und PVPP umfasst.

## Beschreibung

Die Erfindung betrifft ein Verfahren zur Extraktion von Nukleinsäuren mittels einem Nukleinsäuren-Extraktionsmaterial, welches mindestens ein Ionenaustauschmaterial und PVPP umfasst. Weiterhin betrifft die Erfindung die Verwendung des Extraktionsmaterials und einen Kit zur Nukleinsäuren-Extraktion.

Die Polymerase Kettenreaktion (PCR *Polymerase chain reaction*) ist eine der wichtigsten biochemischen Methoden zur Amplifikation von Nukleinsäuren. Mit dieser Technik können Nukleinsäuren sehr schnell und effektiv amplifiziert und anschließend sequenziert oder nachgewiesen werden. Ein wichtiges Einsatzgebiet der PCR ist die klinische Diagnostik. Hier wird die Technik unter anderem für den Nachweis von Krankheitserregern wie Noroviren eingesetzt. Ein anderes Einsatzgebiet ist die Lebensmittelanalytik. So ist es zum Beispiel möglich, in Lebensmittelproben Kontaminationen mit Salmonellen oder die Anwesenheit von Allergenen nachzuweisen.

Ein weiterer Bereich der PCR ist die Analyse hinsichtlich pathogener Mikroorganismen in Lebensmitteln. So beschreibt beispielweise die EP 0 643 140 B1 ein Verfahren zur Bestimmung von Nukleinsäuren durch den Nachweis des Amplifikationsprodukts der PCR unter Verwendung einer Farbstoffverbindung. Der Farbstoff fluoresziert nicht im freien Zustand. Erst bei der Umsetzung mit einer doppelsträngigen Nukleinsäure kann eine Fluoreszenz festgestellt werden. Hierdurch ist es möglich, die Art und Anzahl von Bakterien in einer Probe zu bestimmen.

Pathogene Mikroorganismen, die mittels PCR-Verfahren in Lebensmitteln nachgewiesen werden sind beispielsweise *Listeria monocytogenes*, *Campylobacter jejuni* und Salmonellen. Mit pathogenen Mikroorganismen kontaminierte Nahrung sorgt für Erkrankungen wie Salmonellose oder Listeriose. Regelmäßige Kontrollen der Produktchargen sind notwendig, damit keimbelastete Lebensmittel nicht in den Handel gelangen. Der konventionelle mikrobielle *Salmonella*-Nachweis in Lebensmitteln benötigte durch lange Anreicherungszeiten mehr als 3 Tage. Die real-time PCR als sensitive Nachweismethode bietet dahingegen den Vorteil, dass ein Ergebnis schon nach einem Tag feststeht. Die Analyse von Lebensmitteln wie Milch, Milchprodukten und Eiern sowie Eierzeugnisse und Fleisch ist unerlässlich. Weiterhin ist es wichtig, neben Bakterien auch bestimmte Viren in Lebensmitteln zu detektieren. Noroviren gelten als Erreger akuter Gastroenteritiden. Die Noroviren gehören in die Familie der *Calciviridae* und sind einzelsträngige, hüllenlose RNA-Viren die weltweit verbreitet sind. Zu dem charakteristischen Krankheitsbild einer akuten Gastroenteritis gehört starke Übelkeit, gefolgt von plötzlichem Erbrechen und Durchfällen, selten mit Fieber. Nach 12 bis 60 Stunden ist das Virus aus dem Körper ausgeschieden. Eine Therapie erfolgt rein symptomatisch und besteht aus ausreichender Zufuhr von Flüssigkeit und Elektrolyten. Die Noroviren werden über den Stuhl und das Erbrochene ausgeschieden. Der Stuhl enthält dabei eine besonders hohe Viruskonzentration, für eine Infektion ist eine Dosis mit weniger als 100 Viruspartikeln ausreichend. Das Virus ist hochinfektiös und umweltstabil. Die Ansteckung erfolgt fäkal-oral, zum Beispiel durch Handkontakt mit kontaminierten Flächen oder durch orale Aufnahme virushaltiger Tröpfchen bei starkem Erbrechen. Der Nachweis von Noroviren kann einerseits mit einem ELISA oder über die Detektion von Nukleinsäuren erfolgen. Der Nachweis mit der PCR-Technik macht eine reverse Transkriptase notwendig. Die transkriptierte RNA wird als cDNA (*complementary Desoxyribonucleic acid*) vervielfältigt und detektiert. Der Nachweis von Noroviren mittels RT-PCR ist deutlich sensitiver und spezifischer als der Nachweis mit einem ELISA.

Für die Untersuchung von Lebensmittelproben oder sonstigen Proben mit Hilfe der PCR ist eine genaue Probenvorbereitung und Nukleinsäureaufreinigung sowie Entfernung der Inhibitoren von großer Bedeutung, da inhibitorische Substanzen den Erfolg der PCR beeinträchtigen in dem sie die Amplifikation unterdrücken, die Taq-Polymerase inaktivieren oder das Messsignal unterdrücken.

Die Aufreinigung von Nukleinsäuren an festen Phasen, die auf einer Silicamatrix basieren, ist eine in vielen kommerziellen Kits angewande Technik. Das Prinzip der Reinigung beruht auf der Bindung der Nukleinsäuren an der Festphase in Abhängigkeit vom pH-Wert und von der Salzkonzentration des Puffers. Unter chaotropen Bedingungen wird das Netzwerk der Wasserstoff-Brücken-Beziehungen im Wasser gestört. Dadurch wird die Ausbildung einer Hydrathülle um die Makromoleküle (DNA, RNA) aufgehoben. In Abwesenheit der chaotropen Ionen bildet sich nochmals eine Hydrathülle aus, so dass die Interaktion zwischen Silicamembran und Makromolekül aufgehoben wird. Technisch wurde diese Art der Aufreinigung einerseits in der Spin-Filter Methode und andererseits in der *magnetic beads* Technologie umgesetzt.

In der WO 1996/09404 ist beispielsweise eine Nukleinsäure-Extraktion offenbart. Prinzipiell erfolgt die Extraktion in vier Schritten: Zelllyse, binden der Nukleinsäuren an eine Matrix, sowie waschen und eluieren der Nukleinsäuren. Nachteilig hierbei ist, dass die Extraktion sehr zeitaufwendig ist, da z. B. zahlreiche Waschschritte durchgeführt werden müssen, bei denen immer Nukleinsäure mit ausgewaschen wird. Hierdurch wird der Ertrag der Nukleinsäure erheblich reduziert. Des Weiteren ist diese Extraktionsmethode nur schwer automatisierbar.

Eine weitere Variante zur Isolierung von Nukleinsäuren mit einer Silicamatrix ist beispielsweise in der WO 1998/031840 offenbart, bei der magnetische Silicapartikel verwendet werden. Dabei werden Moleküle mit einer großen Oberfläche eingesetzt die ein magnetisches Moment besitzen, wenn sie einem Magnetfeld ausgesetzt sind. Zum Einsatz kommen unter anderem mit kolloidalem Magnetit (Fe₃O₄) oberflächenmodifizierte poröse Gläser. Diese *magnetic beads* und ein spezieller *binding buffer* werden nach der Lyse der Probe zugesetzt. Die Nukleinsäuren binden an der Silicamatrix. Durch Anlegen eines magnetischen Feldes sammeln sich die *beads* am Gefäßrand und die Verunreinigungen können in mehreren Waschschritten entfernt werden. Durch Entfernen des Magneten und Zugabe des *elution buffer* lösen sich die Zielmoleküle. Wird ein erneutes Magnetfeld angelegt, kann der *elution buffer* mit Nukleinsäuren von den *beads* getrennt werden. Der Vorteil dieser Technik besteht in der hohen Automatisierbarkeit der Arbeitsabläufe mit einem geringen apparativen Aufwand.

Im Stand der Technik sind weiterhin Verfahren beschrieben, mit deren Hilfe Bakterien in Proben nachgewiesen werden können. So beschriebt beispielsweise die EP 1 574 584 B1 ein Verfahren zur Isolierung von Bakterien aus biologischen Proben. Die Verfahren eignen sich zur Probenvorbereitung biologischer Proben für auf Nukleinsäure beruhende oder immundiagnostische Verfahren zum Nachweis von Bakterien. Hierbei werden Bakterien mittels einem spezifischen Antikörper detektiert und isoliert.

Nachteilig bei dem Stand der Technik ist, dass durch die zahlreichen Aufreinigungsschritte auch die Menge der zu isolierende Nukleinsäure reduziert und somit der Ertrag erheblich verringert wird. Die zahlreichen Aufreinigungsschritte führen weiterhin dazu, dass die Extraktion sehr lange dauert. Außerdem sind die im Stand der Technik beschrieben Verfahren nicht oder nur schwer automatisierbar. Weiterhin kann die Extraktion nicht mit allen Proben durchgeführt werden. Hochkomplexe Proben, die zahlreiche Bestandteile aufweisen, wie beispielsweise Stuhlproben, sind nur schwer zu bearbeiten, da die Nukleinsäure häufig bei dem Aufreinigungsverfahren beschädigt wird.

Aufgabe der Erfindung war es demgemäß, eine Nukleinsäurenextraktion bereitzustellen, die nicht die Nachteile oder Mängel des Stands der Technik aufweist.

Gelöst wird die Aufgabe durch die unabhängigen Ansprüche. Vorteilhafte Ausführungsformen ergeben sich aus den Unteransprüchen.

Es war völlig überraschend, dass ein Nukleinsäuren-Extraktionsmaterial bereitgestellt werden kann, welches nicht die Nachteile des Stands der Technik aufweist und mindestens Polyvinylpolypyrrolidon (PVPP) und ein Ionenaustauschmaterial umfasst. Das erfindungsgemäße Extraktionsmaterial ermöglicht eine schnelle und materialsparende Extraktion oder Aufreinigung von Nukleinsäuren. Das Extraktionsmaterial kann auch vorteilhafterweise für Automatisierungsprozesse genutzt werden. Außerdem ist es universell einsetzbar und muss nicht an individuelle Anwendungen angepasst werden.

Das Ionenaustauschmaterial ist bevorzugt ausgewählt aus der Gruppe umfassend Sephadex, Chelex, Zeolithe und/oder Sepharose. Sephadex beschreibt im Sinne der Erfindung insbesondere ein dreidimensional vernetztes Polysaccharid, das durch Quervernetzung der linearen Makromoleküle von Dextran erhalten wird. Sephadex ist indifferent gegenüber Kationen und Anionen und enthält viele Hydroxy-Gruppen, wodurch es stark hydrophil ist und in Wasser oder einer ElektrolytLösung aufquillt. In einer bevorzugten Ausführungsform kann Sephadex mit funktionellen Gruppen, umfassend Diethylaminoethyl-, Diethyl-2-hydroxypropylaminoethyl-, Carboxymethyl- oder Sulfopropyl-Gruppen konjugiert sein. Sephadex ist vorteilhafterweise ausgewählt aus der Gruppe umfassend Sephadex G-10, Sephadex G-100, Sephadex G-15, Sephadex G-25, Sephadex G-50 und/oder Sephadex G-75. Chelex bezeichnet im Sinne der Erfindung ebenfalls ein Polymer, was bevorzugt Ionen bindet. Zeolithe sind im Sinne der Erfindung kristalline Alumosilicate. Die Kristallgitter der Zeolithe sind insbesondere aus SiO₄- und AlO₄-Tetraedern aufgebaut, die jeweils über Sauerstoff-Brücken miteinander verknüpft sind und bevorzugt Ringe oder Prismen bilden. Diese wiederum verbinden sich zu weiteren sekundären Baueinheiten (secondary building units, SBU), die jeweils bis zu 16 Si- oder Al-Atome enthalten können, woraus eine große Strukturvielfalt folgt. Dabei entsteht eine räumliche Anordnung gleichgebauter Hohlräume, die über Fenster (Porenöffnungen) bzw. dreidimensionale Kanalsysteme zugänglich sind. Es hat sich gezeigt, dass unterschiedliche Elemente, umfassend Ionen, Proteine, Kohlenhydrate oder Fette mit Zeolithe wechselwirken und von Nukleinsäuren trennbar sind. Es kann weiterhin vorteilhaft sein, Sepharose als Ionenaustauschmaterial zu verwenden. Sepharose bezeichnet im Sinne der Erfindung ein modifiziertes Polysaccharid auf Agarose Basis, deren Polysaccharid-Ketten zu einem dreidimensionalen Netzwerk verknüpft sind.

PVPP bezeichnet im Sinne der Erfindung insbesondere Polyvinylpolypyrrolidone (auch Crospovidon, abgeleitet von cross-linked Polyvinylpyrrolidon), was insbesondere beim Erhitzen von Vinylpyrrolidinon mit Alkalien oder Divinyl-Verbindungen entsteht. PVPP ist ein vernetztes Polymer, was im Wesentlichen in Wasser und allen Lösemitteln unlöslich ist. PVPP ist vorteilhafterweise ein aus Vinylmonomeren bestehendes Copolymer. Es kann bevorzugt sein, anstelle von PVPP oder in Kombination mit PVPP ein weiteres aus Vinylmonomeren bestehendes Copolymer für ein bevorzugtes Nukleinsäuren-Extraktionsmaterial zu verwenden. Ein aus Vinylmonomeren bestehendes Copolymer weist bevorzugt die allgemeine Formel 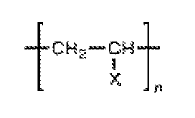 auf. Hierbei steht X für ein Heteroatom oder eine über ein Heteroatom fixierte Gruppe. Im der folgenden Tabelle sind bevorzugte Gruppen dargestellt:

**Tabelle 1:**

| | |
|---|---|
| **X** | **Name** |
| -OH | Polyvinylalkohol (aus Polyvinylacetat) |
| -Halogen | Polyvinylchlorid, Polyvinylfluorid usw. |
| -O-CO-R | Polyvinylester |
| 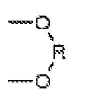 | Polyvinylacetale |
| -O-R | Polyvinylether |
| -P(O)(OH)₂ | Polyvinylphosphonsäuren |
| -C₆H₅ | Polystyrol |
| -SO₃H | Polyvinylsulfonsäuren |
| -NH₂ | Polyvinylamine |
| 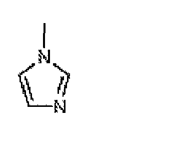 | Polyimidazole |
| 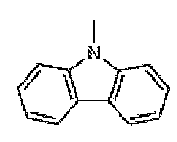 | Polyvinylcarbazole |
| 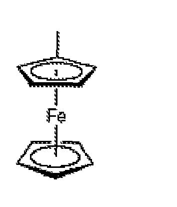 | Polyvinylferrocene |

Die bevorzugten Homo- oder Copolymere umfassen insbesondere Monomere der Struktur H₂C=CH-X, die aus einer polymerisierbaren Vinyl-Gruppe und einem Substituenten X bestehen, der seinerseits aus nur einem einzigen Atom [z. B. F (Vinylfluorid), Cl (Vinylchlorid), Br (Vinylbromid)] oder einer Atomgruppe bestehen kann. Beispiele für letztere sind X = Alkyl (1-Alkene), Aryl (zum Beispiel Styrol), OR (Vinylether), O-CO-R (Vinylester), COOR (Acrylsäure und deren Ester), CONR₂ (Acrylamide), CN (Acrylnitril), NR₂ (Vinylamine), NH-CO-R (Vinylamide), SO₃H (Vinylsulfonsäure), PO(OH)₂ (Vinylphosphonsäure) und andere. Auch Monomere mit der Struktureinheit C=C-C=C, also 1,3-Diene, sind im Wesentlichen Vinylmonomere. Sie werden aber auch als Dienmonomere bezeichnet. Darüber hinaus gehören auch Di- und Polyvinylmonomere wie z. B. das Divinylbenzol zu den Vinylmonomeren.

Im weiteren Sinne werden auch andere Monomere mit C,C-Doppelbindungen, z. B. die des Typs H₂C=CR¹R², z. B. Vinylidenchlorid (R¹ = R² = Cl), im Sinne der Erfindung zu den Vinylmonomeren gerechnet. Schließlich sind auch Verbindungen R¹R²C=CR³R⁴ mit nur einem oder schließlich gar keinem direkt an die ungesättigten Kohlenstoff-Atome gebundenem Wasserstoff-Atom substituierte Vinylmonomere.

Das Extraktionsmaterial kann vorteilhafterweise als loses Pulver, Tablette, Pellet oder Chromatographiesäulenfüllmaterial vorliegen, welches mindestens einen Ionenaustauschmaterial- und einen PVPP-Anteil umfasst. Es ist bevorzugt, dass das lose Pulver, die Tablette, das Pellet oder das Chromatographiesäulenfüllmaterial den lonenaustauschmaterial- und den PVPP-Anteil in einer Korngrößenfraktion von 5-1000µm, bevorzugt in einem Bereich von 50-250µm, enthalten.

Es war völlig überraschend, dass mittels des erfindungsgemäßen Extraktionsmaterials, Bestandteile aus Proben gebunden werden können, wobei die zu isolierende Nukleinsäure nicht bindet. Vorteilhafterweise wird das Extraktionsmaterials zur Probenaufbereitung, insbesondere zur Aufbereitung von Proben, bevorzugt Nukleinsäureproben zur PCR-Analyse verwendet.

Eine Probe ist bevorzugt ein Kulturmedium, eine Körperflüssigkeit und/oder eine Mischung aus Material pflanzlichem und/oder tierischen Ursprungs. Überraschenderweise können auch Nukleinsäuren aus Lebensmittelproben extrahiert werden. Dem Fachmann ist bekannt, dass Körperflüssigkeiten z. B. Stuhl, Speichel, Blut, Lymphe, Urin, Synovialflüssigkeit, Verdauungssäfte, Sekrete, Exkrete oder Flüssigkeitsausscheidung oder weitere Flüssigkeiten eines Organismus umfassen. Die Probe kann in flüssiger oder fester Form vorliegen, wobei eine feste Form im Sinne der Erfindung insbesondere auch gefrorene Proben oder Gewebe- oder Knochenproben umfasst. Dem Fachmann ist bekannt, dass Mikroorganismen vorzugsweise in einem Kulturmedium kultiviert werden, wobei das Kulturmedium alle für Mikroorganismen relevanten Nährstoffe beinhaltet. Das Kulturmedium kann in fester oder flüssiger Form vorliegen und dient insbesondere der Kultur von Mikroorganismen umfassend Bakterien, Archaea, Pilze, Mikroalgen, Protozoen und Viren.

Die Nukleinsäure oder die Nukleinprobe umfasst bevorzugt RNA und/oder DNA. RNA bezeichnet ein aus Nucleotiden bestehendes langgestrecktes Molekül, das in der Zelle hauptsächlich die Funktion hat, die in der Desoxyribonucleinsäure (DNA) gespeicherte genetische Information umzusetzen. Daran sind verschiedene Formen der RNA beteiligt, die mRNA, die als Abschrift der Gene die Information zur Protein-Biosynthese (Translation) liefert, die rRNA die in Form verschiedener Spezies (5S, 16S, 23S in Bakterien und 5S, 5,8S, 18S, 28S in höheren Organismen) in den Ribosomen vertreten ist, und die tRNA, die den Einbau der aktivierten Aminosäuren in die wachsende Protein-Kette an den Ribosomen vermittelt. Im Zellkern findet sich noch die aus Vorstufen der mRNA bestehende heterogene Kern-RNA (hnRNA von heterogeneous nuclear RNA), wie auch die beim Zusammenfügen der Exons der RNA mitwirkende kleine Kern-RNA (snRNA). RNA-Moleküle können auch enzymatische Aktivitäten besitzen (Ribozyme) oder regulatorische Funktion durch RNA-Interferenz (siRNA, miRNA) übernehmen. Solche RNAs faßt man häufig unter dem Begriff ncRNA zusammen. Bei RNA-Viren ist die RNA selbst Träger der genetischen Information. Es war völlig überraschend, dass mittels einem bevorzugten Extraktionsmaterial RNA umfassend mRNA, tRNA, rRNA, snRNA, miRNA, Viren-RNA oder hnRNA aus einer Probe extrahiert werden kann. Vorteilhafterweise kann ebenso DNA aus einer Probe isoliert werden.

DNA bezeichnet im Sinne der Erfindung insbesondere langkettige Polynucleotide, welche die hauptsächliche genetische Information (das Genom) der Lebewesen in sich gespeichert enthalten. Die Hauptmenge der DNA ist bei Eukaryonten im Zellkern enthalten, und zwar in den Chromosomen bzw. im Chromatin. Bei Bakterien befindet sie sich nicht in einer separaten Zellorganelle und besteht meist aus einem einzelnen, ringförmig geschlossenen Molekül. Bakterien enthalten neben der genomischen DNA kleinere, ebenfalls ringförmige DNA-Moleküle, die leicht übertragbaren Plasmide. Es war völlig überraschend, dass mittels des Nukleinsäuren-Extraktionsmaterials DNA einfach und schnell isoliert werden kann. Die DNA wird im Wesentlichen nicht beschädigt und kann so im Ganzen einer nachfolgenden Analyse zugeführt werden. Dies stellt einen erheblichen Vorteil gegenüber dem Stand der Technik dar, da eine Beschädigung der DNA nur eine unvollständige Analyse zulässt. Mittels des Extraktionsmaterials sind umfangreichere Analysen durchführbar.

Die Erfindung umfasst mithin auch ein Verfahren zur Aufreinigung einer in einer Probe vorliegenden Nukleinsäure in einem analytischen oder präparativen Maßstab, umfassend eine Matrix umfassend
a. ein synthetisches oder natürliches Ionenaustauschmaterial und
b. ein vernetztes Polymer mit einer Pyrrolidonstruktur,
wobei die Probe mit der Matrix in Kontakt gebracht wird und insbesondere unerwünschte Bestandteile der Probe, umfassend Proteine, Salze, Kohlenhydrate, Ionen und/oder Fette mit der Matrix wechselwirken. Hierdurch ist eine schnelle Extraktion der Nukleinsäure möglich, wobei die Trennung, Aufreinigung und/oder Detektion der Nukleinsäure bevorzugt in einem Verfahrensschritt durchgeführt wird und vorteilhafterweise keine Wasch- und/oder Elutionsschritte notwendig sind. Das Verfahren zur Probenaufbereitung zur Extraktion von Nukleinsäuren aus einer Probe, umfasst folgende Schritte:
- in Kontakt bringen der Probe mit einem Lysepuffer,
- Erhitzen des Gemisches aus Probe und Lysepuffer,
- Zentrifugieren des Gemisches,
- Aufnahme des Überstandes und
- in Kontakt bringen des Überstandes mit dem Nukleinsäure-Extraktionsmaterial.

Mittels dem Verfahren können vorteilhafterweise aus Proben, bevorzugt Körperflüssigkeiten, pflanzliche oder tierische Proben und/oder Kulturmedien Nukleinsäuren extrahiert werden. So ist es überraschenderweise mögliche, aus Stuhlproben bakterielle oder virale Nukleinsäuren, aus pflanzlichen oder tierischen Proben Allergieassoziierte Nukleinsäuren und/oder aus Kulturmedienproben Nukleinsäuren zu extrahiert. Es ist keine Bindung der Nukleinsäure an eine Membran oder sonstige Matrix notwendig, wodurch keine Wasch- und Elutionsschritte durchgeführt werden müssen und hierdurch der Ertrag der isolierten Nukleinsäure maßgeblich verbessert werden kann. Außerdem kann das Verfahren einfach in kleinem oder großem Maßstab auch voll- oder teilautomatisch durchgeführt werden.

Die zu untersuchende Probe wird in einem ersten Schritt mit einem Lysepuffer in Kontakt gebracht. Eine Lyse bezeichnet im Sinne der Erfindung insbesondere eine Auflösung (Lysis) von Zellen unter Mitwirkung lytischer Enzyme (Lysozyme) und eine Zerstörung der Zellmembranen (Cytolyse). Es kann jedoch auch bevorzugt sein, dass die Probe mittels mechanischer Verfahren, wie Ultraschallbehandlung, French Press oder Glasperlenmühle aufgeschlossen wird. Bevorzugt ist jedoch ein Lysepuffer, da hierdurch die Nukleinsäuren weniger beschädigt werden. Der Lysepuffer umfasst in einer bevorzugten Ausführungsform einen Puffer, der einen bevorzugten pH-Bereich einstellt.

Es ist bevorzugt, dass der Lysepuffer Triton, bevorzugt Triton X-15, X-35, X-45, X-100, X-102, X-104, X-114, X-165, X-305, X-405 und/oder X-705, umfasst. Triton ist ein nichtionisches Tensid aus der Gruppe der Octylphenolethoxylate, welches Proteine denaturiert. Tenside sind amphiphile (bifunktionelle) Verbindungen mit mindestens einem hydrophoben und einem hydrophilen Molekülteil. Der hydrophobe Rest ist zumeist eine möglichst lineare Kohlenwasserstoff-Kette mit bevorzugt 8 bis 22 Kohlenstoff-Atomen. Der hydrophile Rest ist entweder eine negativ oder positiv elektrisch geladene (hydratisierbare) oder eine neutrale polare Kopfgruppe. Grenzflächenaktive Betaine oder Aminosäure-Tenside (amphotere oder zwitterionische Tenside) tragen negativ und positiv geladene Gruppen in einem Molekül. Vorteilhafte Eigenschaften der Tenside sind die orientierte Adsorption an Grenzflächen sowie die Aggregation zu Micellen und die Ausbildung von lyotropen Phasen. Nichtionische Tenside weisen eine ungeladene, wasserlöslich machende Kopfgruppe auf.

Es war völlig überraschend, dass durch den Puffer, dem Triton zugesetzt wurde, der Nukleinsäure-Extraktionsertrag maßgeblich verbessert wird. Außerdem weist die aufgereinigte Nukleinsäure eine hohe Reinheit auf, d. h. sie ist im Wesentlichen durch keine weiteren Bestandteile verunreinigt. Die Reinheit der Nukleinsäure spielt insbesondere bei weiteren Analyseverfahren eine wichtige Rolle, da zahlreiche Enzyme, wie beispielsweise die DNA-Polymerase, durch Verunreinigungen in ihrer Funktion beeinträchtigt werden. Hierdurch kann es zu falschen Ergebnissen kommen. Der Fachmann weiß, dass die Anzahl und Verfügbarkeit von biologischen Proben häufig begrenzt sind und jede Probe mit Vorsicht zu benutzen ist. Demgemäß kann das erfindungsgemäße Extraktionsmaterial als ein technischer Fortschritt angesehen werden, da die Nukleinsäure während der Extraktion nicht beschädigt wird und somit ein optimales Ausgangsmaterial für nachfolgende Analysen darstellt. Es sind außerdem keine weiteren Fällungsreaktionen notwendig, um weitere Verunreinigungen zu entfernen - die extrahierte Nukleinsäure kann in dieser Form weiterverwendet werden.

Nach der Zugabe des Lysepuffers kann es bevorzugt sein, das Gemisch aus dem Lysepuffer und der Probe zu erwärmen oder zu erhitzen, um eine vollständige Lyse der zellulären Bestandteile sicherzustellen. Es hat sich als überaus vorteilhaft herausgestellt, das Gemisch, bevorzugt Stuhl auf 80°C bis 95°C, bevorzugt 90°C für 5 Minuten bis 15 Minuten, besonders bevorzugt für 7 bis 10 Minuten zu erwärmen/erhitzen. Bei den bevorzugten Temperaturen wird im Wesentlichen eine vollständige Denaturierung der proteinogenen Bestandteile der Probe erreicht, wobei die Nukleinsäure nicht beschädigt wird.

Nach dem Erhitzen wird die Probe bevorzugt zentrifugiert, wobei sich bei Raumtemperatur eine Triton-Phase bildet, die sowohl ungelöste Probenbestandteile sowie unpolare Bestandteile umfasst. Weiterhin bildet sich vorteilhafterweise eine wässrige Phase - ein Überstand - in der die Nukleinsäure vorliegt. Dieser Überstand kann beispielsweise auf einen Zentrifugen-Filter aufgetragen und zentrifugiert werden. Ein bevorzugter Filter ist beispielsweise ein Spin-Filter. Der Filter umfasst in einer bevorzugten Ausführungsform der Erfindung das Nukleinsäure-Extraktionsmaterial, insbesondere PVPP und ein Ionenaustauschmaterial. Die Nukleinsäure durchdringt den Filter und liegt in Lösung vor, wobei alle inhibierenden Bestandteile durch das Ionenaustauschmaterial und PVPP gebunden werden und den Filter nicht durchdringen können. Es kann bevorzugt sein, die Nukleinsäure nach Trennung, Aufreinigung und/oder Detektion aufzukonzentrieren. Die Aufkonzentrierung kann beispielsweise mittels Vakuumverdampfung oder Fällung erfolgen.

In einer bevorzugten Ausführungsform liegt das Extraktionsmaterial als Tablette, Pellet oder Chromatographiesäulenfüllmaterial vor, welches mindestens einen Ionenaustauschmaterial- und einen PVPP-Anteil umfasst. Es war völlig überraschend, dass das Extraktionsmaterial als Tablette ausgeführt werden kann. Hierdurch ist ein leichter Transport und Lagerung des Extraktionsmaterials möglich. Vorteilhafterweise erfolgt das in-Kontakt-bringen so, dass der Überstand mit einer Tablette, umfassend PVPP und Ionenaustauschmaterial, in Kontakt gebracht wird und dass das so gewonnene Gemisch wiederum zentrifugiert und der Überstand insbesondere für eine PCR-Reaktion verwendet wird. Nach der Lyse der Probe kann die Tablette zu dem Überstand hinzugefügt werden. Hierdurch ist eine schnelle Extraktion der Nukleinsäure möglich. Es kann weiterhin bevorzugt sein, dass der Lysepuffer und/oder Ionenaustauschmaterial als Tablette vorliegen. Der Lysepuffer in Tablettenform wird der Probe zugeführt, wobei sich die Tablette auflöst und die Probe lysiert. Die Ionenaustauschmaterial-Tablette kann vorteilhafterweise in den Überstand mit dem PVPP eingebracht werden.

Es kann jedoch auch bevorzugt sein, dass das in-Kontakt-bringen des Überstandes mit dem Nukleinsäure-Extraktionsmaterial so erfolgt, dass der Überstand auf eine Chromatographiesäule, umfassend das Nukleinsäure-Extraktionsmaterial, aufgebracht wird, und dass das mittels der Säule gewonnene Eluat in einem PCR-Verfahren verwendet wird. Chromatographie bezeichnet im Sinne der Erfindung insbesondere die physikalisch-chemische Trennung von Substanzgemischen aufgrund ihrer unterschiedlichen Verteilung zwischen einer stationären und einer mobilen Phase. Die Einzelkomponenten werden in Folge verschieden starker Wechselwirkungen mit der stationären Phase in Bezug zur mobilen Phase unterschiedlich verzögert; sie legen somit in gleicher Zeit unterschiedliche Strecken zurück. Bevorzugt Chromatographien sind z. B.: Dünnschichtchromatographie, Gaschromatographie, Hochleistungs- oder Hochdruckflüssigkeitschromatographie (HPLC), lonenchromatographie und Säulenflüssigkeitschromatographie. Durch die Chromatographie kann eine schnelle und effiziente Aufreinigung der Probe erreicht werden, da das Nukleinsäuren-Extraktionsmaterial, umfassend Ionenaustauschmaterial und PVPP, in der Säule als Chromatographiesäulenfüllmaterial vorliegt. Hierdurch ist auch die Reinheit der extrahierten Nukleinsäure nach der Chromatgraphie sehr hoch. Im Wesentlichen können alle inhibierenden oder störenden Bestandteile aus der Probe durch die Chromatographie von der Nukleinsäure getrennt werden. Die Bestandteile werden bevorzugt in der Säule zurückbehalten, wobei die Nukleinsäure durch die Säule hindurchtritt.

Falls lediglich eine bestimmte Nukleinsäure analysiert werden soll, kann durch die Zugabe von Enzymen ein Verdau stattfinden. Wenn nur RNA analysiert werden soll, kann die DNA durch DNasen verdaut und abgetrennt werden. Umgekehrt kann die RNA durch RNasen verdaut werden, falls nur die DNA analysiert werden soll.

In einer bevorzugten Ausführungsform wird das Extraktionsmaterial zur Probenaufbereitung, insbesondere zur Aufbereitung von Nukleinsäureproben zur PCR-Analyse verwendet. Der Fachmann weiß, dass eine Aufbereitung von Nukleinsäureproben einer Extraktion von Nukleinsäuren aus einer Probe entspricht. Eine PCR umfasst bevorzugt long range PCR, nested PCR, inverse PCR, anchored PCR, RT-PCR und quantitative RT-PCR (auch real-time PCR).

Insbesondere bei der PCR-Analyse ist es wichtig, dass inhibierende Bestandteile aus den Proben entfernt werden, da die PCR ansonsten in ihrer Funktionsweise beeinträchtigt wird. PCR-Inhibitoren werden nach ihrer Wirkungsweise grundsätzlich in drei Kategorien eingeteilt: (1) Inaktivierung der DNA-Polymerase, (2) Abbau der Nukleinsäuren und (3) die negative Beeinflussung der Lyse. Inhibierend wirkende Substanzen umfassen Salze, Ionen (z. B. Calciumionen), Fette oder Proteine. Allerdings zeigen auch Verbindungen die zur der Probenvorbereitung eingesetzt werden, inhibierende Wirkung wie zum Beispiel Ethanol oder Detergenzien wie Natriumdodecylsulfat. Es war völlig überraschend, dass inhibierende Bestandteilen einfach und schnell von der Nukleinsäure getrennt werden können. Vorteilhafterweise muss die Nukleinsäure nicht durch zahlreiche Elutionsschritte von einer Matrix eluiert werden, sondern die Bestandteile werden gebunden und die Nukleinsäure liegt bevorzugt in einer Lösung vor.

Die Abtrennung oder Bindung der Bestandteile, die ggf. eine nachfolgende PCR inhibieren können, erfolgt insbesondere durch zwei Mechanismen. Der erste Mechanismus umfasst die Bindung von unpolaren Verbindungen in einer wässrigen Phase der Probe insbesondere durch PVPP, dass bei definierten Bedingungen eine nichtwässrige Phase bildet. Durch den zweiten Mechanismus werden Inhibitoren aus der wässrigen Phase durch eine Abtrennung durch Molekulargewichtsunterschiede mittels des Ionenaustauschmaterials entfernt.

Vorteilhafterweise kann eine mittels des Extraktionsmaterials extrahierte Nukleinsäure auch durch andere Analysen- oder Quantifizierungsmethoden charakterisiert werden. Es kann beispielsweise bevorzugt sein, die extrahierte Nukleinsäure einem Restriktionsverdau zu unterziehen, bei dem die Nukleinsäure an definierten Positionen von Restriktionsenzymen geschnitten wird. Die zerschnittene Nukleinsäure kann anschließend beispielsweise durch eine Gelelektrophorese analysiert werden. Auch die Analyse der extrahierten Nukleinsäure mittels Microarray-Methoden oder sonstigen High-Throughput-Screening-Verfahren ist bevorzugt.

Die Erfindung betrifft weiterhin einen Kit zur Nukleinsäuren-Extraktion, der mindestens ein Ionenaustauschmaterial und PVPP umfasst. Mit dem Kit können Nukleinsäuren einfach und schnell aus einer Probe extrahiert werden. Vorteilhafterweise kann der Kit noch einen Lysepuffer insbesondere in Tablettenform umfassen. Auch das Ionenaustauschmaterial kann in Tablettenform vorliegen.

Die Erfindung führt zu zahlreichen Vorteilen umfassend:
- es kann mehr Nukleinsäure extrahiert werden, da diese nicht an eine Matrix bindet und durch unvollständige Bindung Nukleinsäure verlorengeht;
- keine Diskriminierung zwischen verschiedenen Bestandteilen der Probe;
- einfache und schnelle Durchführung mit reduzierte Anzahl an Arbeitsschritten;
- Automatisierung möglich;
- Geringe Kosten für das Extraktionsmaterial, da keine Wasch- oder Elutionslösungen benötigt werden und das
- Extraktionsmaterial hat einen einfachen Aufbau - lediglich PVPP und ein lonenaustauschmaterial sind nötig.

Die Erfindung kann ebenfalls als Kombinationserfindung angesehen werden. Die Kombination von den bekannten Elementen Ionenaustauschmaterial und PVPP führt zu überraschenden Effekten bei der Extraktion von Nukleinsäuren. Erst die Kombination dieser Verbindungen führt zu synergistischen Vorteilen, die eine effiziente Trennung der inhibierenden Bestandteile von den Nukleinsäuren bewirken. Die Bestandteile binden an die Kombination von Ionenaustauschmaterial und PVPP, wobei die Nukleinsäuren nicht mit dem Ionenaustauschmaterial oder PVPP interagieren und somit leicht von den Bestandteilen abgetrennt werden können.

## Patentansprüche

1. Nukleinsäuren-Extraktionsmaterial, umfassend mindestens Polyvinylpolypyrrolidon (PVPP) und ein Ionenaustauschmaterial.

2. Extraktionsmaterial nach dem vorhergehenden Anspruch,
**dadurch gekennzeichnet, dass**
das das Ionenaustauschmaterial ausgewählt ist aus der Gruppe umfassend Sephadex, Chelex, Zeolithe und/oder Sepharose.

3. Extraktionsmaterial nach einem oder mehreren der vorhergehenden Ansprüche,
**dadurch gekennzeichnet, dass**
es als loses Pulver, Tablette, Pellet oder Chromatographiesäulenfüllmaterial vorliegt, welches mindestens einen lonenaustauschmaterial- und einen PVPP-Anteil umfasst.

4. Extraktionsmaterial nach einem oder mehreren der vorhergehenden Ansprüche,
**dadurch gekennzeichnet, dass**
das lose Pulver, die Tablette, das Pellet oder das Chromatographiesäulenfüllmaterial den lonenaustauschmaterial- und den PVPP-Anteil in einer Korngrößenfraktion von 5-1000µm, bevorzugt in einem Bereich von 50-250µm, enthalten.

5. Verwendung des Extraktionsmaterials nach einem der vorhergehenden Ansprüche zur Probenaufbereitung, insbesondere zur Aufbereitung von Proben zur PCR-Analyse.

6. Verwendung nach dem vorhergehenden Anspruch,
**dadurch gekennzeichnet, dass**
die Probe ein Kulturmedium, eine Körperflüssigkeit und/oder eine Mischung aus Material pflanzlichem und/oder tierischen Ursprungs ist.

7. Ein Verfahren zur Aufreinigung einer in einer Probe vorliegenden Nukleinsäure in einem analytischen oder präparativen Maßstab, umfassend eine Matrix umfassend
a. ein synthetisches oder natürliches Ionenaustauschmaterial und
b. ein vernetztes Polymer mit einer Pyrrolidonstruktur,
wobei die Probe mit der Matrix in Kontakt gebracht wird und insbesondere unerwünschte Bestandteile der Probe, umfassend Proteine, Salze, Kohlenhydrate, Ionen und/oder Fette mit der Matrix wechselwirken.

8. Verfahren nach Anspruch 7, wobei die Aufreinigung der Nukleinsäure in einem Verfahrensschritt durchgeführt wird.

9. Verfahren zur Extraktion von Nukleinsäuren aus einer Probe nach einem oder mehreren der vorherigen Ansprüche,
**dadurch gekennzeichnet, dass**
das Verfahren folgende Schritte umfasst:
- in Kontakt bringen der Probe mit einem Lysepuffer,
- Erhitzen des Gemisches aus Probe und Lysepuffer,
- Zentrifugieren des Gemisches,
- Aufnahme des Überstandes und
- in Kontakt bringen des Überstandes mit dem Nukleinsäureextraktionsmaterial nach Anspruch 1 bis 4.

10. Verfahren nach dem vorhergehenden Anspruch
**dadurch gekennzeichnet, dass**
das in Kontakt bringen des Überstandes mit dem Nukleinsäureextraktionsmaterial so erfolgt, dass der Überstand auf eine Chromatographiesäule, umfassend das Nukleinsäureextraktionsmaterial, aufgebracht wird, und dass das mittels der Säule gewonnene Eluat in einem PCR-Verfahren verwendet wird.

11. Verfahren nach einem der vorhergehenden Ansprüche
**dadurch gekennzeichnet, dass**
das in Kontakt bringen so erfolgt, dass der Überstand mit einer Tablette, umfassend PVPP und Ionenaustauschmaterial, in Kontakt gebracht wird und dass das so gewonnene Gemisch wiederum zentrifugiert und der Überstand für eine PCR-Reaktion verwendet wird.

12. Verfahren nach einem der vorhergehenden Ansprüche,
**dadurch gekennzeichnet, dass**
der Lysepuffer Triton, bevorzugt Triton X-15, X-35, X-45, X-100, X-102, X-104, X-114, X-165, X-305, X-405 und/oder X-705 umfasst.

13. Verfahren nach einem der vorhergehenden Ansprüche,
**dadurch gekennzeichnet, dass**
das Erwärmen/Erhitzen des Gemisches aus dem Lysepuffer und der Probe, bevorzugt Stuhl bei 80°C bis 95°C, bevorzugt bei 90°C für 5 Minuten bis 15 Minuten, besonders bevorzugt für 7 bis10 Minuten erfolgt.

14. Verfahren nach einem der vorhergehenden Ansprüche,
**dadurch gekennzeichnet, dass**
die Probe Körperflüssigkeiten, pflanzliche oder tierische Proben und/oder Kulturmedien umfasst.

15. Verfahren nach einem der vorhergehenden Ansprüche,
**dadurch gekennzeichnet, dass**
die Nukleinsäure nach Trennung, Aufreinigung und/oder Detektion aufkonzentriert wird.

16. Kit zur Nukleinsäuren-Extraktion, umfassend mindestens ein lonenaustauschmaterial und PVPP.
